# EUROPEAN PATENT APPLICATION

(11) **EP 2 520 300 A1**
(43) Date of publication of application: **07.11.2012**
(21) Application number: 10840974.9
(22) Date of filing: 27.12.2010
(51) Int. Cl.: A61K 31/496, A61K 9/12, A61K 9/20, A61P 3/06, A61P 9/10, A61P 43/00

(54) **PHARMACEUTICAL COMPOSITION FOR ORAL ADMINISTRATION**

(30) Priority: 29.12.2009 US 290813 P
(71) Applicant: Kowa Co., Ltd., Nagoya-shi, Aichi 460-8625 (JP)
(72) Inventor: KOZAKI, Masato, Fuji-shi Shizuoka 417-8650 (JP); SHIMOKAWA, Tatsuharu, Fuji-shi Shizuoka 417-8650 (JP); TANIZAWA, Yoshio, Fuji-shi Shizuoka 417-8650 (JP)
(74) Representative: Blodig, Wolfgang
(86) International application number: PCT/JP2010/073498
(87) International publication number: WO 2011/081118

(57) **Abstract**

The present invention relates to a solid pharmaceutical composition for oral administration, containing the following components (A) and (B):
2-[4-[2-(benzimidazol-2-ylthio)ethyl]piperazin-1-yl]-N-[2,4 -bis(methylthio)-6-methyl-3-pyridyl]acetamide (compound a) or an acid addition salt thereof, and (B) citric acid. An improvement is achieved in the dissolubility of the compound a, which is useful as a therapeutic agent for hypercholesterolemia, arteriosclerosis, and the like.

## Description

### FIELD OF THE INVENTION

The present invention relates to a pharmaceutical composition for oral administration having enhanced dissolubility.

### BACKGROUND OF THE INVENTION

2-[4-[2-(Benzimidazol-2-ylthio)ethyl]piperazin-1-yl]-N-[2,4-bis(methylthio)-6-methyl-3-pyridyl]acetamide (hereinafter, referred to as compound a) or an acid addition salt thereof is known to have an excellent acyl-coenzyme A: cholesterol acyltransferase (ACAT) inhibitory action, and an excellent intracellular cholesterol transport inhibitory action, and to be useful as a therapeutic agent for hypercholesterolemia, arteriosclerosis and acute myocardial infarction (Patent Documents 1 and 2).

### PRIOR ART DOCUMENTS

### PATENT DOCUMENT

Patent Document 1: WO 1998/054153
Patent Document 2: WO 2005/020996

### SUMMARY OF THE INVENTION

### PROBLEM TO BE SOLVED BY THE INVENTION

Most of the patients of hypercholesterolemia, arteriosclerosis and the like are elderly people, and the treatment of these diseases requires long times periods. Therefore, as the therapeutic agents for these diseases, pharmaceutical compositions for oral administration that can be stably administered for a long time period are desirable. When conventional pharmaceutical compositions for oral administration are orally administered, active medicinal ingredients rapidly dissolve out from the compositions in the upper digestive tract such as the stomach, and are first absorbed, and the effect of a drug occurs.
However, compound a has low solubility in weakly acidic environments to alkaline environments (for example, the solubility in water is 0.05% (W/V)). Therefore, in the case of patients of achlorhydria, which is considered to develop more frequently in elderly people, there has been a concern that the dissolubility of the compound a from the pharmaceutical composition in the stomach may be insufficient, and there may be variations in the efficacy of the drug.
Accordingly, an object of the present invention is to provide a solid pharmaceutical composition for oral administration containing the compound a, which has improved dissolubility in the digestive tract.

### SOLUTION TO PROBLEM

Thus, the inventors of the present invention conducted extensive investigations on the improvement of the dissolubility of a solid composition containing a compound a, and as a result, the inventors found that among various additives, acidic substances improve the dissolubility of the compound a. Thus, the inventors further conducted an investigation, and as a result, they found that among various acidic substances, citric acid has an especially excellent effect of improving the dissolubility of the compound a. Furthermore, the inventors also found that when a disintegrant is used in combination with this, the dissolubility of the compound a is further enhanced, thus completing the present invention.

That is, the present invention is to provide a solid pharmaceutical composition for oral administration containing the following components (A) and (B): (A) 2-[4-[2-(benzimidazol-2-ylthio)ethyl]piperazin-1-yl]-N-[2,4 -bis(methylthio)-6-methyl-3-pyridyl]acetamide (compound a) or an acid addition salt thereof, and (B) citric acid.
The present invention is to provide a composition as described above, containing component (B) in an amount of more than 0.2 parts by mass relative to 1 part by mass of the compound a in component (A).
Furthermore, the present invention is to provide a composition as described above, in which the mass ratio of the compound a in component (A) the component (B), (A/B) is 10/3 to 1/20.
Furthermore, the present invention is to provide a composition as described above, further containing (C) a disintegrant.
Furthermore, the present invention is to provide a composition as described above, in which the disintegrant (C) is selected from the group consisting of crospovidone, croscarmellose sodium, pregelatinized starch, partly pregelatinized starch, and sodium carboxymethyl starch.
Furthermore, the present invention is to provide a composition as described above, in which the disintegrant (C) is crospovidone or pregelatinized starch.
Moreover, the present invention is to provide a method for treating hypercholesterolemia or arteriosclerosis, the method including orally administering a solid pharmaceutical composition containing the compound a or an acid addition salt thereof, and citric acid.

### EFFECTS OF INVENTION

Since the solid pharmaceutical composition of the present invention has satisfactory dissolubility of the compound a in the digestive tract, the solid pharmaceutical composition exhibits a rapid and stabilized therapeutic effect on hypercholesterolemia, arteriosclerosis and the like by oral administration.

### BRIEF DESCRIPTION OF DRAWINGS

FIG. 1 shows the results of a dissolution test in Test Example 1;
FIG. 2 shows the results of a dissolution test in Test Example 3;
FIG. 3 shows the results of a dissolution test in Test Example 5; and
FIG. 4 shows the results of a dissolution test in Test Example 7.

### DESCRIPTION OF EMBODIMENTS

The compound a (A), which is an active ingredient of the pharmaceutical composition of the present invention, is known to have an excellent ACAT inhibitory action and an excellent intracellular cholesterol transport inhibitory action, as described in the Patent Document 1, and to be useful as a therapeutic agent for hypercholesterolemia, arteriosclerosis and the like. Examples of the acid addition salt of the compound a include addition salts of inorganic acids such as hydrochloric acid, sulfuric acid and nitric acid; and addition salts of organic acids such as acetic acid, lactic acid and succinic acid, and among these, a hydrochloride is preferred. Furthermore, the compound a may also be in the form of a hydrate. Further, the compound a can be prepared by the preparation method described in the Patent Document 1.

The content of the compound a in the pharmaceutical composition of the present invention is preferably 10 mg to 300 mg, and more preferably 25 mg to 200 mg, from the viewpoint of therapeutic effects. There are no particular limitations on the particle size of the compound a to be used; however, from the viewpoints of dissolubility, absorbability and the like, an average particle size of 0.1 µm to 200 µm is preferred, and an average particle size of 1 µm to 150 µm is more preferred.

The pharmaceutical composition of the present invention contains (A) compound a and (B) citric acid. Citric acid has an action of remarkably improving the dissolubility of the compound a from the pharmaceutical composition. As the citric acid, any of citric acid anhydride and citric acid hydrate may be used, but it is preferable to use citric acid anhydride. As commercially available products thereof, citric acid anhydride of the Japanese Pharmacopoeia (fine powder), citric acid hydrate of the Japanese Pharmacopoeia (manufactured by Komatsuya Corp.), citric acid anhydride (fine granules), citric acid hydrate (citric acid (crystals)) (fine granules) (manufactured by DSM Nutrition Japan K.K.), and the like are available. Furthermore, the average particle size of citric acid is preferably 1000 µm or less, and particularly preferably 500 µm or less, from the viewpoint of the effect of improving the dissolubility of the compound a.

The content of citric acid is preferably an amount of more than 0.2 parts by mass, and more preferably 0.3 parts by mass or greater, relative to 1 part by mass of the compound a, from the viewpoint of the dissolubility of the compound a. Furthermore, there are no particular limitations on the upper limit of the content of citric acid, but in view of the preparation of solid compositions, it is preferable to set the upper limit to 20 parts by mass or less, and more preferably 5 parts by mass or less, relative to 1 part by mass of the compound a. Therefore, in the pharmaceutical composition, the mass ratio of the compound a in component (A) to (B) citric acid, (A/B) is preferably set in the range of 5/1 to 1/20, more preferably in the range of 10/3 to 1/20, and even more preferably in the range of 2/1 to 1/5.

When the pharmaceutical composition of the present invention further contains (C) a disintegrant in addition to (A) compound a and (B) citric acid, the dissolubility of the compound a is further markedly improved. Examples of such a disintegrant include crospovidone, croscarmellose sodium, pregelatinized starch, partly pregelatinized starch, sodium carboxymethyl starch, carmellose, carmellose sodium, carmellose calcium, low-substituted hydroxypropyl cellulose, and hydroxypropyl starch.
Among these, crospovidone, croscarmellose sodium, pregelatinized starch, partly pregelatinized starch, and sodium carboxymethyl starch are particularly preferred.

Crospovidone is a crosslinked polymer of N-vinyl-2-pyrrolidone. As for crospovidone, it is preferable to use a product having an average particle size of 5 µm to 100 µm. Croscarmellose sodium is a crosslinked polymer of carmellose sodium. Pregelatinized starch is a product obtained by pregelatinizing starch and water by heating, and then rapidly drying the pregelatinized product. Partly pregelatinized starch is a product obtained by heating corn starch together with water under normal pressure or under pressure to partially pregelatinize starch grains, and then drying the pregelatinized product. Sodium carboxymethyl starch (also referred to as sodium starch glycolate) is a sodium salt of carboxymethyl ether of starch.
Among these (C) disintegrants, it is particularly preferable to use crospovidone or pregelatinized starch from the viewpoint of the effect of improving the dissolubility of the compound a. As commercially available products of crospovidone, for example, POLYPLASDONEXL, POLYPLASDONE XL-10, POLYPLASDONE INF-10 (manufactured by ISP Japan, Ltd.), KOLLIDON CL, KOLLIDON CL-F, KOLLIDON CL-SF, and KOLLIDON CL-M (manufactured by BASF Japan, Ltd.) are available. Furthermore, as commercially available products of pregelatinized starch, for example, SWELSTAR PD-1 (manufactured by Asahi Kasei Chemicals Corp.), LYCATAB PGS (manufactured by Roquette Japan K.K.), and AMICOL (manufactured by Nippon Starch Chemical Co., Ltd.), and the like are available.

The content of the (C) disintegrant is preferably 0.1 to 1 part by mass, and more preferably 0.2 to 0.8 parts by mass, relative to 1 part by mass of the component a.

The pharmaceutical composition of the present invention is a solid composition for oral administration, and specific examples thereof include tablets, granules, fine granules, capsules, powders, and pills. However, among these, tablets, granules and capsules are preferred, and tablets are particularly preferred in view of ingestability.

The solid composition of the present invention can have an excipient, a binder, a lubricant and the like added thereto, in addition to the compound a, citric acid and a disintegrant, and can be formulated into the respective forms. Examples of the excipient include lactose, corn starch, crystalline cellulose, sucrose, glucose, mannitol, sorbitol, and calcium carbonate. Examples of the binder include hydroxypropyl cellulose, hydroxyethyl cellulose, hypromellose, hydroxyethylethyl cellulose, hydroxyethylmethyl cellulose, polyvinylpyrrolidone, and polyvinyl alcohol. Examples of the lubricant include magnesium stearate, stearic acid, palmitic acid, calcium stearate, and talc.

When the dissolubility of the compound a is considered, the contents of the excipient, binder and lubricant in the pharmaceutical composition of the present invention are preferably set to 0.2 to 4 parts by mass for the excipient, 0.05 to 1 part by mass for the binder, and 0.01 to 0.08 parts by mass for the lubricant, relative to 1 part by mass of the compound a.

There are no particular limitations on the method for preparing the solid pharmaceutical composition of the present invention, but for example, in the case of tablets, the tablets may be produced by uniformly mixing the various components described above, and producing the tablets by a general-purpose wet granulation compression method, a direct powder compression method, or the like. Furthermore, the tablets thus obtained may be further subjected to film coating, sugar coating, sustained release coating, or the like. In this case, examples of the coating agent include hypromellose, hydroxypropyl cellulose, polyvinyl alcohol, titanium oxide, talc, polyethylene glycol, triethyl citrate, stearic acid, hydrated silicon dioxide, and light silicic anhydride. Examples of the sugar coating include gum arabic, purified gelatin, gelatin, purified sucrose, sucrose, precipitated calcium carbonate, talc, and calcium dihydrogen phosphate hydrate. Examples of the sustained release coating agent include methacrylic acid copolymer LD, ethyl cellulose, aminoalkyl methacrylate copolymer RS, and hypromellose.

Despite the fact that the compound a has low solubility in water, the pharmaceutical composition of the present invention has markedly improved dissolubility of the compound a from the composition, as a result of the addition of citric acid. The reason for this is not clearly known, but it can be speculated that when the compound a is brought into contact with water, the incorporation of citric acid causes a decrease in the pH in the vicinity of the compound a in the microscopic scale. However, as it is obvious from the comparisons made between Examples and Comparative Examples below, if the reason is merely a decrease in the pH, the dissolubility of the compound a will also be improved by adding salicylic acid or phthalic anhydride. However, since a satisfactory effect of improving dissolubility cannot be obtained in the case of these organic acids, it can be contemplated that certain factors other than pH are involved. That is, the effect of the present invention can be considered to be unique to the combination of the compound a and citric acid.

### EXAMPLES

Next, the present invention will be described in detail by way of Examples, but the present invention is not intended to be limited to these.

The following Examples were carried out using monohydrochloride of compound a (hereinafter, referred to as compound a hydrochloride). Furthermore, the compound a hydrochloride was synthesized by using the method described in Patent Document 1 and known methods.

### Example 1

53.65 mg of compound a hydrochloride (50 mg in terms of compound a) and 50 mg of citric anhydride were pulverized and mixed in a mortar, and thus a sample 1 (103.65 mg) was obtained.

### Comparative Example 1

53.65 mg of compound a hydrochloride was pulverized in a mortar, and thus a sample 2 (53.65 mg) was obtained.

### Comparative Example 2

53.65 mg of compound a hydrochloride and 50 mg of salicylic acid were pulverized and mixed in a mortar, and thus a sample 3 (103.65 mg) was obtained.

### Comparative Example 3

53.65 mg of compound a hydrochloride and 50 mg of sorbic acid were pulverized and mixed in a mortar, and thus a sample 4 (103.65 mg) was obtained.

### Comparative Example 4

53.65 mg of compound a hydrochloride and 50 mg of phthalic anhydride were pulverized and mixed in a mortar, and thus a sample 5 (103.65 mg) was obtained.

### Comparative Example 5

53.65 mg of compound a hydrochloride and 50 mg of boric acid were pulverized and mixed in a mortar, and thus a sample 6 (103.65 mg) was obtained.

### Test Example 1 Dissolution test

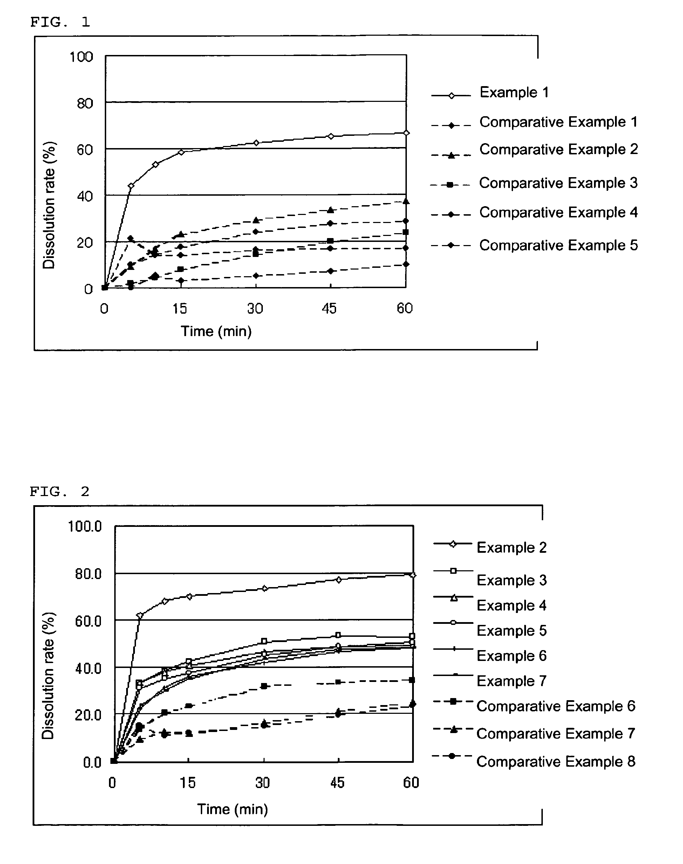

The dissolubility of the samples 1 to 6 was examined according to the second method (paddle method) of the dissolution test method according to the general test methods of the Japanese Pharmacopoeia.
103.65 mg of each of the samples 1 to 6 (53.65 mg for sample 2 only) was introduced into 900 mL of the second fluid for dissolution test according to the Japanese Pharmacopoeia, and the test was carried out under the conditions of a temperature of 37 ± 0.5°C and a speed of paddle rotation of 50 rpm. Thus, the concentrations of the compound a after 5, 10, 15, 30, 45 and 60 minutes were measured. The sample solution collected at each time point was filtered through a membrane filter made of PTFE (DISMIC-25HP manufactured by Toyo Roshi Kaisha, Ltd.) having a pore size of 0.45 µm, and was analyzed by a high performance liquid chromatographic method using a reversed phase column (manufactured by Nomura Chemical Co., Ltd.; Develosil ODS-HG-5). Thus, the dissolution rates were calculated. The results are presented in Table 1 and FIG. 1.

### Test Example 2 Measurement of pH value

103. 65 mg of each of the samples 1 to 6 (53.65 mg for sample 2 only) was introduced into a beaker, and 50 mL of purified water was added thereto. While the mixture was stirred with a magnetic stirrer, the pH of the solution was measured with a glass electrode type hydrogen ion concentration meter (manufactured by Toa Electronics, Inc.; HM-50V). 50 mL of purified water was further added thereto, and then the measurement of pH was carried out in the same manner. The results are presented in Table 1.

**[Table 1]**

| Time (min) | Example 1 | Comparative Example 1 | Comparative Example 2 | Comparative Example 3 | Comparative Example 4 | Comparative Example 5 |
|---|---|---|---|---|---|---|
| 0 | 0 | 0 | 0 | 0 | 0 | 0 |
| 5 | 44.2 | 0 | 9.1 | 2.2 | 10.0 | 21.4 |
| 10 | 53.0 | 5.7 | 16.8 | 4.5 | 14.3 | 13.9 |
| 15 | 58.5 | 3.1 | 23.3 | 8.1 | 17.5 | 14.0 |
| 30 | 62.4 | 5.2 | 29.2 | 14.3 | 24.0 | 16.6 |
| 45 | 65.2 | 7.1 | 33.8 | 20.0 | 27.5 | 16.7 |
| 60 | 66.4 | 10.2 | 37.2 | 23.5 | 28.3 | 16.9 |
| pH (50mL) | 2.95 | 5.02 | 2.88 | 3.72 | 3.12 | 5.02 |
| pH (100mL) | 3.13 | 5.07 | 2.98 | 3.75 | 3.22 | 5.07 |

As can be seen from the above results, when compound a was used alone, the compound a hardly dissolved, and an improvement of the dissolution rate was not recognized even in the mixture with an organic acid other than citric acid, or with boric acid. However, only the mixture with citric acid exhibited a dissolution rate value of greater than 40% after 30 minutes, which is the criterion of determination.

### Example 2

53.65 mg of compound a hydrochloride and 100 mg of citric anhydride were pulverized and mixed in a mortar, and thus a sample 7 (153.65 mg) was obtained.

### Example 3

53.65 mg of compound a hydrochloride and 45 mg of citric anhydride were pulverized and mixed in a mortar, and thus a sample 8 (98.65 mg) was obtained.

### Example 4

53.65 mg of compound a hydrochloride and 40 mg of citric anhydride were pulverized and mixed in a mortar, and thus a sample 9 (93.65 mg) was obtained.

### Example 5

53.65 mg of compound a hydrochloride and 35 mg of citric anhydride were pulverized and mixed in a mortar, and thus a sample 10 (88.65 mg) was obtained.

### Example 6

53.65 mg of compound a hydrochloride and 25 mg of citric anhydride were pulverized and mixed in a mortar, and thus a sample 11 (78.65 mg) was obtained.

### Example 7

53.65 mg of compound a hydrochloride and 15 mg of citric anhydride were pulverized and mixed in a mortar, and thus a sample 12 (68.65 mg) was obtained.

### Comparative Example 6

53.65 mg of compound a hydrochloride and 10 mg of citric anhydride were pulverized and mixed in a mortar, and thus a sample 13 (63.65 mg) was obtained.

### Comparative Example 7

53.65 mg of compound a hydrochloride and 5 mg of citric anhydride were pulverized and mixed in a mortar, and thus a sample 14 (58.65 mg) was obtained.

### Comparative Example 8

53.65 mg of compound a hydrochloride and 2 mg of citric anhydride were pulverized and mixed in a mortar, and thus a sample 15 (55.65 mg) was obtained.

### Test Example 3 Dissolution test

The dissolubility of the samples 7 to 15 was examined by carrying out the test in the same manner as that described in Test Example 1, except that 153.65 mg of sample 7, 98.65 mg of sample 8, 93.65 mg of sample 9, 88.65 mg of sample 10, 78.65 mg of sample 11, 68.65 mg of sample 12, 63.65 mg of sample 13, 58.65 mg of sample 14, and 55.65 mg of sample 15 were respectively introduced into 900 mL of the second fluid for dissolution test according to the Japanese Pharmacopoeia. The results are presented in Table 2 and FIG. 2.

### Test Example 4 Measurement of pH value

The pH values of the samples 7 to 15 were measured in the same manner as that used in Test Example 2. Meanwhile 153.65 mg of sample 7, 98.65 mg of sample 8, 93.65 mg of sample 9, 88.65 mg of sample 10, 78.65 mg of sample 11, 68.65 mg of sample 12, 63.65 mg of sample 13, 58.65 mg of sample 14, and 55.65 mg of sample 15 were used. The results are presented in Table 2.

**[Table 2]**

| Time (min) | Example 2 | Example 3 | Example | Example 5 | Example 6 | Example 7 | Comparative Example 6 | Comparative Example 7 | Comparative Example 8 |
|---|---|---|---|---|---|---|---|---|---|
| 0 | 0 | 0 | 0 | 0 | 0 | 0 | 0 | 0 | 0 |
| 5 | 61.9 | 33.5 | 33.5 | 30.9 | 22.5 | 23.8 | 13.8 | 9.5 | 15.4 |
| 10 | 68.0 | 38.5 | 38.0 | 35.1 | 31.5 | 29.9 | 20.7 | 13.1 | 11.2 |
| 15 | 70.0 | 42.5 | 40.8 | 37.6 | 36.0 | 35.0 | 23.4 | 12.0 | 12.0 |
| 30 | 73.5 | 50.9 | 46.4 | 45.0 | 42.1 | 43.5 | 31.8 | 16.7 | 15.1 |
| 45 | 77.0 | 53.2 | 48.5 | 48.8 | 46.6 | 47.6 | 33.3 | 21.4 | 19.9 |
| 60 | 79.1 | 52.8 | 49.2 | 50.2 | 47.9 | 47.8 | 34.3 | 24.9 | 23.3 |
| pH (50mL) | 2.87 | 2.98 | 3.02 | 3.07 | 3.28 | 3.36 | 3.56 | 3.80 | 4.15 |
| pH (100mL) | 3.03 | 3.12 | 3.16 | 3.21 | 3.39 | 3.46 | 3.64 | 3.89 | 4.22 |

As can be seen from the above results, in the mixtures of the compound a and citric acid, as the mixing amount of citric acid increased, the dissolution rate of the compound a also increased. When 15 mg or more of citric acid was mixed with 50 mg of compound a, a dissolution rate value of greater than 40% was obtained after 30 minutes.

### Example 8

1.073 kg of compound a hydrochloride, 1.0 kg of citric anhydride (Komatsuya Corp.; citric anhydride of the Japanese Pharmacopoeia (fine powder)), 0.5 kg of lactose hydrate, and 1.5 kg of crystalline cellulose were mixed in a fluidized bed granulation dryer. A binding liquid prepared in advance by dissolving 0. 3 kg of hydroxypropyl cellulose in 3 kg of purified water was sprayed onto the mixture in a fluidized bed granulation dryer, and the mixture was subjected to granulation, drying, and particle size regulation. Thus, granules were obtained. 47 g of magnesium stearate was added and mixed with the granules thus obtained, and the mixture was compression molded with a rotary type tabletting machine. Thus, tablets which weighed 221 mg per tablet were obtained.

### Example 9

1.073 kg of compound a hydrochloride, 1.0 kg of citric anhydride (Komatsuya Corp.; citric anhydride of the Japanese Pharmacopoeia (fine powder)), 0.5 kg of lactose hydrate, 1.5 kg of crystalline cellulose, and 0.379 kg of crospovidone (ISP Japan, Ltd; POLYPLASDONE XL) were mixed in a fluidized bed granulation dryer. A binding liquid prepared in advance by dissolving 0. 3 kg of hydroxypropyl cellulose in 3 kg of purified water was sprayed onto the mixture in a fluidized bed granulation dryer, and the mixture was subjected to granulation, drying, and particle size regulation. Thus, granules were obtained. 48 g of magnesium stearate was added and mixed with the granules thus obtained, and the mixture was compression molded with a rotary type tabletting machine. Thus, tablets which weighed 240 mg per tablet were obtained.

### Example 10

1.073 kg of compound a hydrochloride, 0.8 kg of citric anhydride (Komatsuya Corp.; citric anhydride of the Japanese Pharmacopoeia (fine powder)), 0.5 kg of lactose hydrate, 1.5 kg of crystalline cellulose, and 0.379 kg of crospovidone (ISP Japan, Ltd; POLYPLASDONE XL) were mixed in a fluidized bed granulation dryer. A binding liquid prepared in advance by dissolving 0.3 kg of hydroxypropyl cellulose in 3 kg of purified water was sprayed onto the mixture in a fluidized bed granulation dryer, and the mixture was subjected to granulation, drying, and particle size regulation. Thus, granules were obtained. 48 g of magnesium stearate was added and mixed with the granules thus obtained, and the mixture was compression molded with a rotary type tabletting machine. Thus, tablets which weighed 230 mg per tablet were obtained.

### Comparative Example 9

1.073 kg of compound a hydrochloride, 0.5 kg of lactose hydrate, and 1.5 kg of crystalline cellulose were mixed in a fluidized bed granulation dryer. A binding liquid prepared in advance by dissolving 0.3 kg of hydroxypropyl cellulose in 3 kg of purified water was sprayed onto the mixture in a fluidized bed granulation dryer, and the mixture was subjected to granulation, drying, and particle size regulation. Thus, granules were obtained. 47 g of magnesium stearate was added and mixed with the granules thus obtained, and the mixture was compression molded with a rotary type tabletting machine. Thus, tablets which weighed 171 mg per tablet were obtained.

### Comparative Example 10

1.073 kg of compound a hydrochloride, 0.5 kg of lactose hydrate, 1.5 kg of crystalline cellulose, and 0.379 kg of crospovidone (ISP Japan, Ltd; POLYPLASDONE XL) were mixed in a fluidized bed granulation dryer. A binding liquid prepared in advance by dissolving 0.3 kg of hydroxypropyl cellulose in 3 kg of purified water was sprayed onto the mixture in a fluidized bed granulation dryer, and the mixture was subjected to granulation, drying, and particle size regulation. Thus, granules were obtained. 48 g of magnesium stearate was added and mixed with the granules thus obtained, and the mixture was compression molded with a rotary type tabletting machine. Thus, tablets which weighed 190 mg per tablet were obtained.

### Test Example 5 Dissolution test

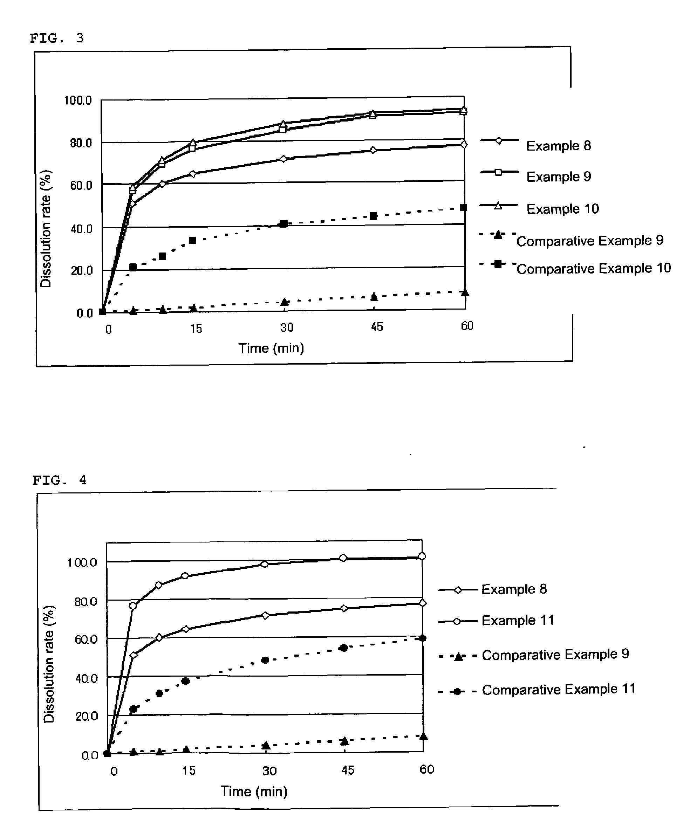

The dissolubility of the tablets of Examples 8 to 10 and Comparative Examples 9 and 10 was examined by carrying out the test by the same method as that described in Test Example 1, except that one tablet each of the Examples and Comparative Examples were introduced into 900 mL of the second fluid for dissolution test according to the Japanese Pharmacopoeia. The results are presented in Table 3 and FIG. 3.

### Test Example 6 Measurement of pH value

The pH values of the tablets of Examples 8 to 10 and Comparative Examples 9 and 10 were measured by the same method as that used in Test Example 2. Meanwhile, one tablet was used for each measurement. The results are presented in Table 3.

**[Table 3]**

| Formulation | Example 8 | Example 9 | Example 10 | Comparative Example 9 | Comparative Example 10 |
|---|---|---|---|---|---|
| Compound a hydrochloride | 53.65 | 53.65 | 53.65 | 53.65 | 53.65 |
| Citric acid anhydride | 50.0 | 50.0 | 40.0 | - | - |
| Lactose hydrate | 25.0 | 25.0 | 25.0 | 25.0 | 25.0 |
| Crystalline cellulose | 75.0 | 75.0 | 75.0 | 75.0 | 75.0 |
| Hydroxypropyl cellulose | 15.0 | 15.0 | 15.0 | 15.0 | 15.0 |
| Crospovidone | - | 18.95 | 18.95 | - | 18.95 |
| Magnesium stearate | 2.35 | 2.4 | 2.4 | 2.35 | 2.4 |
| Total (mg/1 T) | 221 | 240 | 230 | 171 | 190 |

| Time (min) | | | | | |
|---|---|---|---|---|---|
| 0 | 0 | 0 | 0 | 0 | 0 |
| 5 | 51.2 | 56.9 | 59.0 | 0.8 | 21.0 |
| 10 | 60.1 | 69.5 | 71.4 | 1.1 | 26.1 |
| 15 | 64.6 | 76.5 | 79.8 | 1.8 | 33.0 |
| 30 | 71.4 | 85.0 | 88.0 | 4.0 | 40.8 |
| 45 | 74.9 | 91.1 | 92.7 | 6.3 | 44.0 |
| 60 | 77.0 | 92.6 | 93.7 | 8.0 | 47.3 |
| pH (50mL) | 3.00 | 2.99 | 3.08 | 5.93 | 5.51 |
| pH (100mL) | 3.13 | 3.11 | 3.19 | 5.80 | 5.54 |

As is obvious from Table 3 and FIG. 3, even if compound a was formulated (tablet), when the disintegrant crospovidone was absent, the compound a hardly dissolved. However, when crospovidone was added, the dissolution rate increased, but dissolubility was still insufficient. On the other hand, it was found that when citric acid was incorporated, dissolubility was enhanced even without crospovidone, and when citric acid and crospovidone were combined, dissolubility markedly increased.

### Example 11

1.073 kg of compound a hydrochloride, 1.0 kg of citric anhydride (Komatsuya Corp.; citric anhydride of the Japanese Pharmacopoeia (fine powder)), 0.5 kg of lactose hydrate, 1.5 kg of crystalline cellulose, and 0.379 kg of pregelatinized starch (Asahi Kasei Chemicals Corp.; SWELSTAR PD-1) were mixed in a fluidized bed granulation dryer. A binding liquid prepared in advance by dissolving 0.3 kg of hydroxypropyl cellulose in 3 kg of purified water was sprayed onto the mixture in a fluidized bed granulation dryer, and the mixture was subjected to granulation, drying, and particle size regulation. Thus, granules were obtained. 48 g of magnesium stearate was added and mixed with the granules thus obtained, and the mixture was compression molded with a rotary type tabletting machine. Thus, tablets which weighed 240 mg per tablet were obtained.

### Comparative Example 11

1.073 kg of compound a hydrochloride, 0.5 kg of lactose hydrate, 1.5 kg of crystalline cellulose, and 0.379 kg of pregelatinized starch (Asahi Kasei Chemicals Corp.; SWELSTAR PD-1) were mixed in a fluidized bed granulation dryer. A binding liquid prepared in advance by dissolving 0.3 kg of hydroxypropyl cellulose in 3 kg of purified water was sprayed onto the mixture in a fluidized bed granulation dryer, and the mixture was subjected to granulation, drying, and particle size regulation. Thus, granules were obtained. 48 g of magnesium stearate was added and mixed with the granules thus obtained, and the mixture was compression molded with a rotary type tabletting machine. Thus, tablets which weighed 190 mg per tablet were obtained.

### Test Example 7 Dissolution test

The dissolubility of the tablets of Example 11 and Comparative Example 11 was examined by carrying out the test in the same manner as that described in Test Example 1, except that each of tablets of Example 11 and Comparative Example 11 was introduced into 900 mL of the second fluid for dissolution test according to the Japanese Pharmacopoeia. The results are presented in Table 4 and FIG. 4.

### Test Example 8 Measurement of pH value

The pH values of the tablets of Example 11 and Comparative Example 11 were measured in the same manner as that used in Test Example 2. Meanwhile, one tablet was used for each measurement. The results are presented in Table 4.

**[Table 4]**

| Formulation | Example 8 | Example 11 | Comparative Example 9 | Comparative Example 11 |
|---|---|---|---|---|
| Compound a hydrochloride | 53.65 | 53.65 | 53.65 | 53.65 |
| Citric acid anhydride | 50.0 | 50.0 | - | - |
| Lactose hydrate | 25.0 | 25.0 | 25.0 | 25.0 |
| Crystalline cellulose | 75.0 | 75.0 | 75.0 | 75.0 |
| Hydroxypropyl cellulose | 15.0 | 15.0 | 15.0 | 15.0 |
| Pregelatinized starch | - | 18.95 | - | 18.95 |
| Magnesium stearate | 2.35 | 2.4 | 2.35 | 2.4 |
| Total (mg/1 T) | 221 | 240 | 171 | 190 |

| Time (min) | | | | |
|---|---|---|---|---|
| 0 | 0 | 0 | 0 | 0 |
| 5 | 51.2 | 76.6 | 0.8 | 23.2 |
| 10 | 60.1 | 87.6 | 1.1 | 31.1 |
| 15 | 64.6 | 92.0 | 1.8 | 37.1 |
| 30 | 71.4 | 97.6 | 4.0 | 47.9 |
| 45 | 74.9 | 100.4 | 6.3 | 54.2 |
| 60 | 77.0 | 101.3 | 8.0 | 58.9 |
| pH (50mL) | 3.00 | 3.02 | 5.93 | 5.76 |
| pH(100mL) | 3.13 | 3.15 | 5.80 | 5.69 |

As is obvious from Table 4 and FIG. 4, it was found that when pregelatinized starch as one kind of disintegrant was incorporated, as in the case of crospovidone of Test Example 5, dissolubility of the compound a was further enhanced.

As can be seen from the above, the problem of dissolubility was solved by mixing compound a with citric acid. Furthermore, when citric acid and a disintegrant, particularly crospovidone or pregelatinized starch, were combined, the dissolubility of the compound a markedly increased, and satisfactory tablets were obtained.

## Claims

1. A solid pharmaceutical composition for oral administration, comprising the following components (A) and (B): (A) 2-[4-[2-(benzimidazol-2-ylthio)ethyl]piperazin-1-yl]-N-[2,4 -bis(methylthio)-6-methyl-3-pyridyl]acetamide (compound a) or an acid addition salt thereof; and (B) citric acid.

2. The composition according to claim 1, comprising the component (B) in an amount of more than 0.2 parts by mass relative to 1 part by mass of the compound a in the component (A).

3. The composition according to claim 1, wherein the mass ratio of the compound a in the component (A) to the component (B), (A/B) is 10/3 to 1/20.

4. The composition according to any one of claims 1 to 3, further comprising component (C), a disintegrant.

5. The composition according to claim 4, wherein the disintegrant (C) is a component selected from the group consisting of crospovidone, croscarmellose sodium, pregelatinized starch, partly pregelatinized starch, and sodium carboxymethyl starch.

6. The composition according to claim 4, wherein the disintegrant (C) is crospovidone or pregelatinized starch.
